(19) Europäisches Patentamt
European Patent Office
Office européen des brevets

(11) Publication number: **0 323 727 B1**

(12) **EUROPEAN PATENT SPECIFICATION**

(45) Date of publication of patent specification: **01.09.93**

(51) Int. Cl.[5]: **C07D 405/12**, A01N 43/54

(21) Application number: **88312047.9**

(22) Date of filing: **20.12.88**

(54) **Heterocyclic-alkylene quinoxalinyloxyphenoxy propanoate herbicides.**

(30) Priority: **06.01.88 US 141182**

(43) Date of publication of application:
**12.07.89 Bulletin 89/28**

(45) Publication of the grant of the patent:
**01.09.93 Bulletin 93/35**

(84) Designated Contracting States:
**AT BE CH DE ES FR GB GR IT LI LU NL SE**

(56) References cited:
**EP-A- 0 023 785**
**EP-A- 0 288 275**

(73) Proprietor: **UNIROYAL CHEMICAL COMPANY, Inc.**
**World Headquarters**
**Middlebury Connecticut 06749(US)**

(72) Inventor: **Davis, Robert Glenn**
**151 Andren Ave., Apt. No. 39**
**Naugatuck 06770, New Haven, Connecticut(US)**
Inventor: **Bell, Allyn Roy**
**540 Riverside Drive**
**Cheshire 06410, New Haven, Connecticut(US)**
Inventor: **Minatelli, John Adrian**
**71 Minisink Trail Goshen**
**Orange, New York 10924(US)**

(74) Representative: **Browne, Robin Forsythe, Dr. et al**
**Urquhart-Dykes & Lord Tower House Merrion Way**
**Leeds LS2 8PA West Yorkshire (GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

FIELD OF THE INVENTION

This invention is directed to novel heterocyclic-alkylene quinoxalinyloxyphenoxypropanoate compounds which exhibit unexpectedly desirable selective herbicidal activity. In other aspects, this invention is directed to herbicidal compositions comprising such compounds as well as to a method for controlling the growth of plants employing such quinoxalinyl derivatives.

BACKGROUND OF THE INVENTION

The control of undesirable grasses is important in the cultivation of many important broadleaf agricultural species such as soybeans, peanuts and cotton, as well as in the cultivation of many horticultural species. Moreover, the presence of such weeds on noncropped areas may present a fire hazard, or may result in the undesirable drifting of sand or snow or irritation to persons with allergies. Accordingly, it would be beneficial to control the growth of undesirable grasses, particularly in a manner which would allow for the selective control of such plants without concurrent injury to desirable broadleaf crops or vegetation.

Among the classes of compounds which have been employed in the past to control the growth of undesirable vegetation are certain quinoxalinyloxyphenoxy compounds. Thus, Ura et al in U.S. Patent 4,629,493 disclose certain quinoxalinyl compounds, including 6-membered homocyclic ring derivatives such as benzyl 2-[4-(6-chloro-2-quinoxalinyloxy)phenoxy]-propanoate, which are useful as selective herbicides. Similarly, in European Patent Application 46,467, Ura et al show a number of quinoxalinyloxyphenoxy compounds, including a morpholino ester thereof, which are useful as herbicides.

Somewhat similarly, U.S. Patent 4,429,167 to Lee discloses a broad range of 3-alkoxy-4-substituted-phenoxy-2,3-unsaturatedacids, including quinoxalinyl derivatives thereof which are selective herbicides for grasses, while U.S. Patent 4,609,396 to Fawzi shows a variety of quinoxalinyloxy ethers which are useful for controlling grass weeds in broadleaf plants. Chemical Abstracts 98, 198278m (1983) discloses herbicidal glycidyl quinoxalin-2-yloxyphenoxypropionateswhile European Patent Application 206,772 shows quinoxalinyloxybenzyl ester compounds which are useful for the selective control of weeds in rice. Spanish Patent Application 8,603,431 shows herbicidal alkyl quinoxalinyloxyphenoxypropanoates while European Patent Application 113,831 shows selective herbicides which are carbamylethyl esters of quinoxalinyloxyphenoxypropanoic acids. European Patent Application 121,871 shows herbicidal phosphoric acid esters of quinoxalinyloxyphenoxypropanoates while U.S. Patent 4,499,271 shows phosphoric acid esters of quinoxalinyloxyphenoxypropanoates. German Application 3,433,390 shows silane esters of quinoxalinyloxyphenoxypropanoic acid, while Kruger et al in U.S. Patent No. 4,601,748 disclose herbicidal 2-phenoxypropionic acid derivatives of pentitols.

In contrast, U.S. Patent 4,564,381 to Bieringer et al shows a very broad class of compounds, including many quinoxalinyloxyphenoxypropanoic esters which function as plant growth stimulants.

While many of the compounds shown in the above publications exhibit desirable herbicidal activity, it would nevertheless be desirable to possess herbicides which exhibited enhanced selective control of undesirable grasses.

Acccordingly, it is an object of this invention to provide a class of novel compounds which exhibit unexpectedly desirable selective herbicidal activity. It is a further object of this invention to provide a method of controlling undersirable weeds.

These objects and other additional objects will become more fully apparent from the following disclosure and accompanying examples.

DESCRIPTION OF THE INVENTION

In one aspect, this invention is directed to a novel class of compounds having the structural formula:

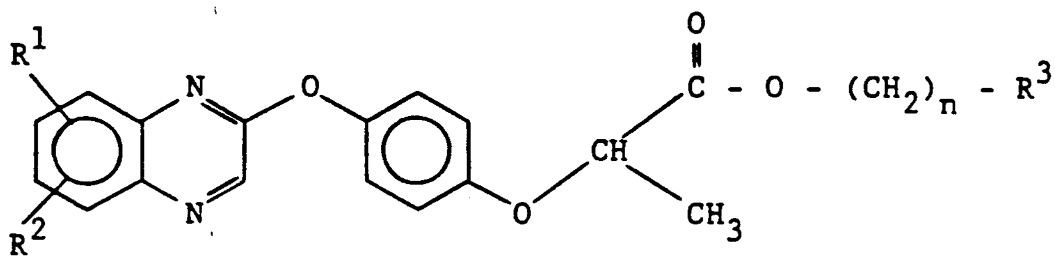

(I)

wherein:

n is 1, 2 or 3;

$R^1$ and $R^2$ are each independently selected from the group consisting of halogen, hydrogen, $C_1$-$C_3$ alkoxy, $C_1$-$C_3$ haloalkoxy, monohalomethyl, dihalomethyl, trihalomethyl, cyanato and nitro; and

$R^3$ is a 5- or 6-membered saturated, unsaturated or partially unsaturated heterocyclic ring containing 1 or 2 oxygen atoms: said ring optionally being substituted with between 1 and 3 substituents each independently selected from the group consisting of oxo, $C_1$-$C_3$ alkyl and $C_1$-$C_3$ alkoxy.

In another aspect, this invention is directed to a herbicidal composition comprised of:

(A) a compound having the structural formula:

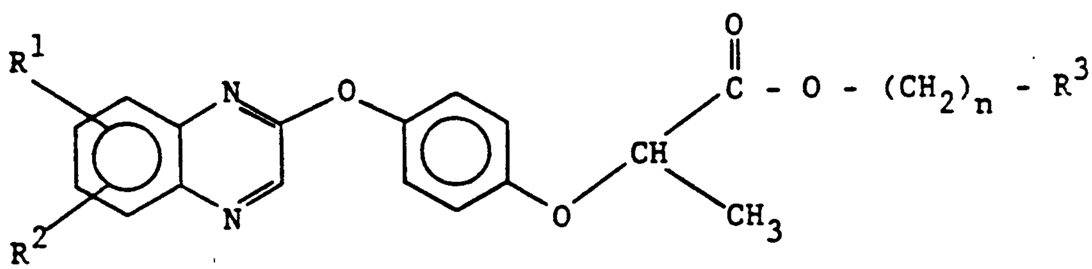

wherein:

n is 1, 2 or 3;

$R^1$ and $R^2$ are each independently selected from the group consisting of halogen, hydrogen, $C_1$-$C_3$ alkoxy $C_1$-$C_3$ haloalkoxy, monohalomethyl, dihalomethyl, trihalomethyl, cyanato and nitro; and

$R^3$ is a 5- or 6-membered saturated, unsaturated or partially unsaturated heterocyclic ring containing 1 or 2 oxygen atoms; said ring optionally being substituted with between 1 and 3 substituents each independently selected from the group consisting of oxo, $C_1$-$C_3$ alkyl and $C_1$-$C_3$ alkoxy, and

(B) a suitable carrier.

In yet another aspect, this invention is directed to a method of controlling undesirable plants, which method comprises applying to the locus of such undesirable plants a herbicidally effective amount of a compound having the structural formula:

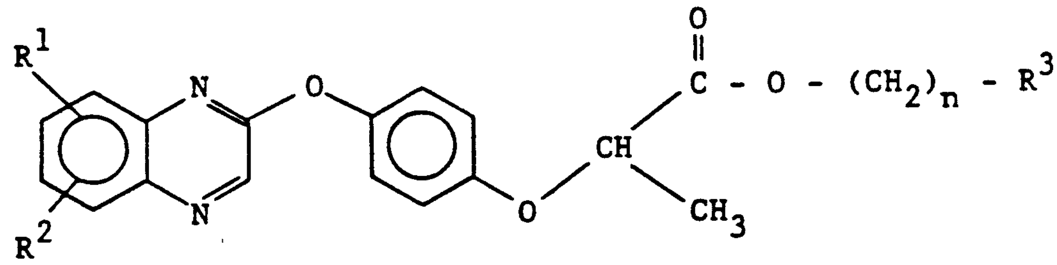

wherein:

- n is 1, 2 or 3;
- $R^1$ and $R^2$ are each independently selected from the group consisting of halogen, hydrogen, $C_1$-$C_3$ alkoxy, $C_1$-$C_3$ haloalkoxy, monohalomethyl, dihalomethyl, trihalomethyl, cyanato and nitro; and
- $R^3$ is a 5- or 6-membered saturated, unsaturated, or partially unsaturated heterocyclic ring containing 1 or 2 oxygen atoms: said ring optionally being substituted with between 1 and 3 substituents each independently selected from the group consisting of oxo, $C_1$-$C_3$ alkyl and $C_1$-$C_3$ alkoxy.

The novel compounds of this invention are of the formula:

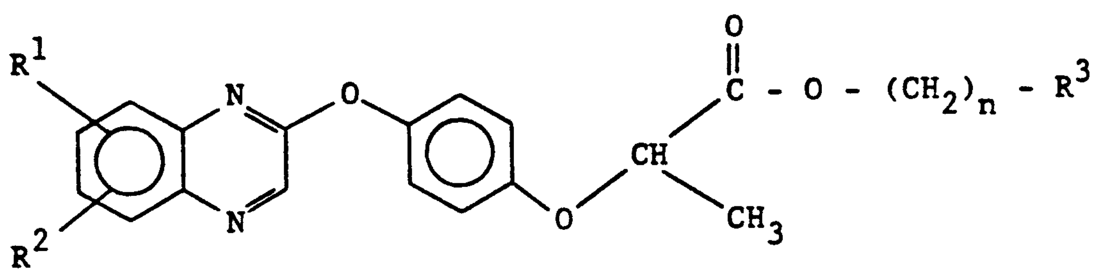

wherein $R^1$, $R^2$, $R^3$ and n are as defined for formula (I) above. It is to be noted that $R^3$ may be an unsaturated. saturated or partially unsaturated (i.e., containing at least one carbon-carbon double bond and at least one carbon-carbon single bond) heterocyclic moiety.

Preferably:

- $R^1$ is chlorine, trichloromethyl or trifluoromethyl;
- $R^2$ is hydrogen; and
- $R^3$ is furanyl, tetrahydrofuranyl, dioxolanyl, tetrahydrodioxolanyl, pyranyl or tetrahydropyranyl; optionally substituted with 1, 2 or 3 moieties selected from the group consisting of oxo and methyl.

Particularly preferred compounds include:

2-tetrahydrofuranylmethyl 2-[4-(6-chloro-2-quinoxalinyloxy)phenoxy]-propanoate;
2-furanylmethyl 2-[4-(6-chloro-2-quinoxalinyloxy)phenoxy]propanoate;
2-tetrahydropyranylmethyl 2-[4-(6-chloro-2-quinoxalinyloxy)phenoxy]-propanoate;
2-[2-(2-methyl-1,3-dioxolanyl)]ethyl 2-[4-(6-chloro-2-quinoxalinyloxy)-phenoxy]propanoate;
4-(3,3-dimethyl-2-butyrolactonyl)methyl 2-[4-(6-chloro-2-quinoxalinyloxy)-phenoxy]propanoate; and
4-(2,2-dimethyl-1,3-dioxolanyl)methyl 2-[4-(6-chloro-2-quinoxalinyloxy)-phenoxy]propanoate.

The compounds of this invention may be prepared by reacting a quinoxalinyloxyphenol compound of the formula:

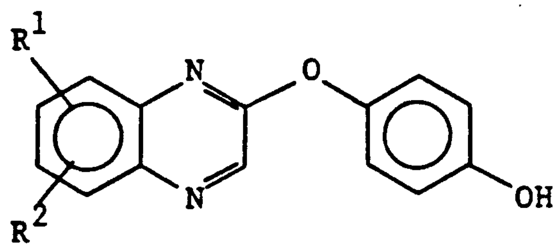

wherein $R^1$ and $R^2$ are as defined for structure (I) above; with a heterocyclicalkylpropanoate halide of the formula:

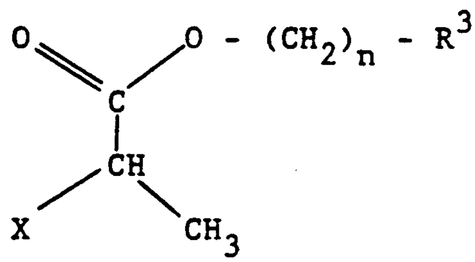

wherein X is halogen, mesylate or tosylate and n and $R^3$ are as defined for structure (I) above. This reaction is typically conducted in an organic solvent (such as dimethylformamide, dimethylsulfoxide, acrylonitrile or the like) in the presence of an inorganic or organic base (such as sodium carbonate, potassium hydroxide or potassium carbonate) at suitable temperature.

The quinoxalinyloxyphenoxy starting materials may be prepared by reacting a 2-haloquinoxaline compound having the formula:

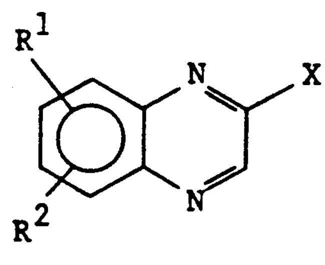

wherein X is halogen and $R^1$ and $R^2$ are as defined in structure (I) above, with a phenyl benzyl ether of the formula:

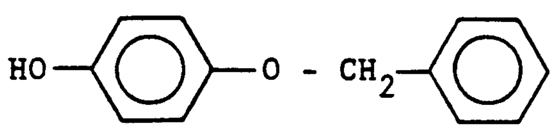

in the presence of an inorganic or organic base to produce an intermediate of the formula:

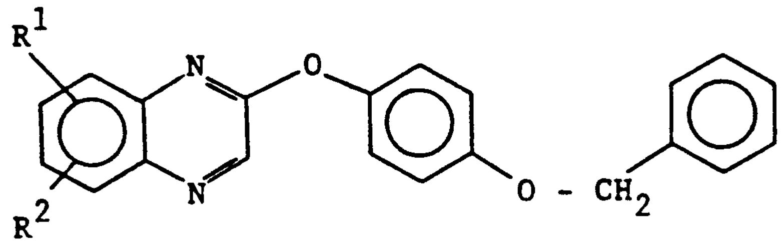

and hydrogenating such intermediate (employing a hydrogenation catalyst such as palladium) to produce a debenzylation thereby forming the quinoxalinyloxyphenol starting material.

The heterocyclicalkylpropanoate halide starting material may be produced by reacting a halogenated propionic acid halide with the appropriate heterocyclic alkyl alcohol.

Alternatively ethyl 2-halopropanoate may be reacted with the appropriate heterocyclic alkyl alcohol in the presence of an appropriate catalyst (such as tetra-isopropoxy titanium) to produce the heterocyclicalkylpropanoate halide via a transesterification process.

The compositions of this invention are comprised of (A) at least one of the quinoxalinyloxyphenoxypropanoate compounds of this invention, and (B) a suitable carrier.

To prepare such herbicidal compositions, the quinoxalinyloxyphenoxypropanoate compound may be mixed with inert ingredients to provide compositions in the form of finely-divided particulate solids, granules, pellets, wettable powders, flowable liquids, soluble powders, solutions, and aqueous or organic solvent dispersions or emulsions. Such formulations may be of several different physical and chemical types, any of which could be made by one familiar with the art. For instance, the active compound may be impregnated on finely-divided or granular inorganic or organic carriers such as attapulgite clay, sand, vermiculite, corn cob, activated carbon or other granular carriers known to the art. The impregnated granules may then be spread on the soil or incorporated into the soil.

Alternatively, the chemical may be formulated as a wettable powder by mixing it with an inactive powdered carrier to which a surface active dispersing agent has been added and grinding the mixture into a fine powder. Typical powdered solid carriers are the various mineral silicates (such as mica, talc, pyrophyllite, clays and the like) or powdered organic material (e.g., corn cob). The wettable powder may then be dispersed in water and sprayed on the soil surface, or on crop or weed plants.

Similarly, an emulsifiable concentrate may be prepared by dissolving the chemical in a solvent such as xylene, toluene, or other aliphatic or aromatic hydrocarbon to which a surface active dispersing agent generally has been added. The emulsifiable concentrate may then be dispersed in water and applied by spraying.

The concentration of active chemical in the composition may vary widely typically ranging from about 1 to about 95% by weight. The concentration of active chemical in dispersions applied to the soil, seed or foliage is typically between about 0.002% and about 80% by weight.

Formulations containing the active ingredient(s) may be dispersed in water or an organic liquid (such as oil) and applied to target plants. Surface active agents may be added to the applied solution to increase its qualitative or quantitive range of activity. Suitable surface active agents are well known to those skilled in the art. Reference may be made to McCutcheon's Detergents and Emulsifiers (1980, Allured Publ. Co., Ridgewood, NJ) for examples of appropriate surface active agents. Similarly, such formulations may be applied to the soil either as a liquid or a granule.

For use as a preemergence herbicide the compound of this invention is typically applied at a rate of from about 0.01 to about 10 pounds per acre (about 0.01 to about 11.2 kg/ha) to soil which contains weed and crop seed. Such application is made either to the surface of the soil or into the upper one to three inches (2.5 to 7.5 cm.) of soil. When employed as a postemergence herbicide the compound is typically applied at a rate of from about 0.01 to about 10 pounds per acre (about 0.01 to about 11.2 kg/ha) to the aerial portions of weeds.

The most suitable dosage of application, and the most effective type and amount of adjuvant substance will depend on a number of factors, including the plant species; the stage of plant development; the method of application; the air and soil temperature and the quantity and intensity of rainfall before and after treatment; the soil type, pH, fertility and moisture and organic matter content; the physiological condition and vigor of the target plants; the relative humidity and wind velocity of the air around the crop at the time

of treatment; the extent and density of the foliar canopy of the target plant; the light quality, intensity and duration each day; the type and interval of previous and subsequent crop protectant chemical applications. However, one skilled in the art can, by routine experimentation, readily determine optimum conditions for the employment of any particular quinoxyalinyloxyphenoxypropanoate compound.

The compounds of the present invention include the isomeric forms and mixtures thereof. Thus, the invention includes the optically active isomers and racemic mixtures. Unless otherwise specified herein, the compounds described in the examples are racemic mixtures.

## Examples

The following Examples are intended to further illustrate the invention and are not intended to limit the scope of the invention in any manner whatsoever.

### Example 1

Preparation of 2-tetrahydrofuranylmethyl 2-[4-(6-chloro-2-quinoxalinyloxy)-phenoxy]propanoate (Compound Number 4).

A. Preparation of 2-tetrahydrofuranylmethyl 2-bromopropanoate

To a 250 milliliter three-necked flask were added 0.15 mole of 2-(hydroxymethyl)tetrahydrofuran, 0.15 mole of triethylamine, and 100 milliliters of diethylether. The flask was immersed in ice, and 0.15 mole of 2-bromopropanoyl chloride introduced dropwise. The ice bath was removed, and the reaction stirred at ambient temperature for two hours. The mixture was extracted with an equal volume of water, and the organic phase dried over sodium sulfate. Decantation and solvent removal resulted in 2-tetrahydrofuranylmethyl 2-bromopropanoate in 94% yield.

B. Preparation of 2-tetrahydrofuranylmethyl 2-[4-(6-chloro-2-quinoxalinyloxy)-phenoxy]propanoate

To a 500 milliliter roundbottom flask were added 0.055 mole of 2-(4-hvdroxyphenoxy)-6-chloroquinoxaline, 0.055 mole of 2-tetrahydrofuranylmethyl 2-bromopropanoate, 0.110 mole of anhydrous potassium carbonate, and 250 milliliters of acetonitrile. The mixture was refluxed for 5.5 hours and the solvent removed. The residue was filtered through a column of alumina with dichloromethane. Solvent removal and recrystallization from boiling hexane resulted in an 85% yield of 2-tetrahydrofuranylmethyl 2-[4-(6-chloro-2-quinoxalinyloxy)phenoxy]propanoate. The material melted over a range of 52-55 °C.

### Example 2

Additional compounds within the scope of the invention were prepared using essentially the procedures outlined above. The structures and melting points and/or NMR spectra of these compounds are summarized in Table I below. (Note: in such NMR data s = singlet: d = duplet; t = triplet; q = quartet; and m = multiplet).

## Table I

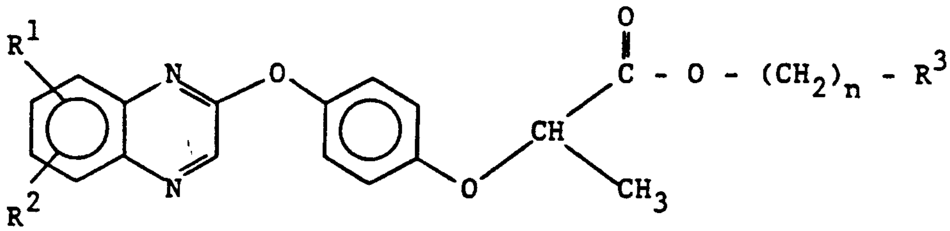

| Compound Number | $R^1$ | $R^2$ | $R^3$ | n | Melting Point (°C) |
|---|---|---|---|---|---|
| 1 | 6-Cl | H | 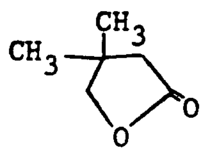 | 1 | 130-134° |
| 2 | 6-Cl | H | 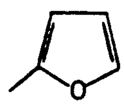 | 1 | 98-101° |
| 3 | 6-Cl | H | 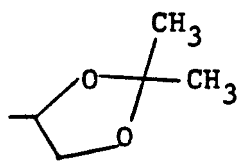 | 1 | Oil* |
| 4 | 6-Cl | H | 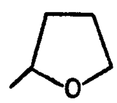 | 1 | 52-55° |
| 5 | 6-Cl | H | 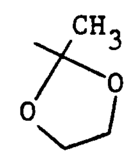 | 2 | Oil** |
| 6 | 6-Cl | H | 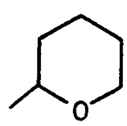 | 1 | 60-64° |

* NMR($CDCl_3$): 1.38(s,3H), 1.43(s,3H), 1.68(d,3H), 3.50-4.24(m,5H), 4.80(q,1H), 7.02(q,4H), 7.59(s,2H), 8.00(s,1H), 8.61(s,1H).

** NMR($CDCl_3$): 1.46(s,3H), 1.77(d,3H), 2.14(t,2H), 3.99(s,4H), 4.38(t,2H), 4,81(q,1H), 7.07(q,4H), 7.62(s,2H), 8.02(s,1H), 8.62(s,1H).

Example 3

Preemergence Control

To illustrate the effectiveness of the heterocyclicalkylene quinoxalinyloxyphenoxypropanoate compounds of this invention as preemergence herbicides, 300 mg of each of the below listed compounds were dissolved in 10 ml acetone to which 30 mg of an emulsifying agent, ethoxylated sorbitan monolaurate, were added. The solution was diluted to 100 ml with distilled water. Ten milliliters of this 3000 ppm solution were diluted to 250 ppm with distilled water. The chemical was applied at the rate of 10 lb/A (11.2 kg/ha) by drenching 46 ml of the 250 ppm solution on the surface of soil in 4-1/2 inch (11.25 cm.) plastic pots wherein seeds of the following weeds had been planted; velvet leaf (Abutilon theophrasti Medic.), "VL"; jimsonweed (Datura stramonium L.), "JW"; tall morningglory (Ipomea purpurea L. Roth), "TM": switchgrass (Panicum virgatum L.), "SG"; barnyard grass (Echinolchloa crus-galli (L.) Beauv.), "BG"; and green foxtail (Setaria viridis) (L.) Beauv.), "GF".

The percent control of the weeds compared to untreated checks was determined two weeks after treatment. The results of such testing are summarized in Table II below.

Table II

| Preemergent Control | | | | | | |
|---|---|---|---|---|---|---|
| Compound Number | Percent Weed Control | | | | | |
| | VL | JW | TM | BG | SG | GF |
| 1 | 0 | 0 | 0 | 100 | 100 | 100 |
| 2 | 0 | 0 | 0 | 100 | 100 | 100 |
| 3 | 0 | 0 | 0 | 100 | 100 | 100 |
| 4 | 0 | 0 | 0 | 100 | 100 | 100 |
| 5 | 0 | 0 | 0 | 100 | 100 | 100 |
| 6 | 0 | 0 | 0 | 100 | 100 | 100 |

The above data shows the excellent selective preemergent herbicidal control exhibited by the compounds of this invention.

Example 4

Postemergence Control

To illustrate the effectiveness of the compounds of this invention as postemergence herbicides, the 3000 ppm solutions described under Example 3 were atomized with a conventional DeVilbiss [trademark] sprayer, wetting the foliage to the drip point. The remainder of the procedure was the same as described under Example 3. The weeds, which were the same species as described under Example 3, were treated six days after emergence.

The percent weed control was evaluated two weeks after treatment. The results of such testing are summarized in Table III below.

Table III

| Postemergence Treatment | | | | | | |
|---|---|---|---|---|---|---|
| Compound Number | Percent Weed Control | | | | | |
| | VL | JW | TM | BG | SG | GF |
| 1 | 0 | 0 | 0 | 100 | 100 | 100 |
| 2 | 0 | 0 | 15 | 100 | 100 | 100 |
| 3 | 10 | 90 | 15 | 100 | 100 | 100 |
| 4 | 0 | 20 | 10 | 100 | 100 | 100 |
| 5 | 0 | 0 | 40 | 100 | 100 | 100 |
| 6 | 0 | 20 | 10 | 100 | 100 | 100 |

The above data shows the desirable selective postemergent herbicidal control exhibited by the novel compounds of this invention.

Example 5

To illustrate the effectiveness of the novel heterocyclicalkylene quinoxalinyloxyphenoxypropanoate derivatives of this invention for preemergence grass control relative to prior art homocyclicalkylene quinoxalinyloxyphenoxypropanoate compounds, solutions of several of the compounds were prepared in a manner similar to that described in Example 1. The 250 ppm solutions were diluted to 3.1 ppm with distilled water to provide testing at a rate of 0.14 kg/ha as a 46 ml drench to 11.25 cm diameter pots. In a similar manner, solutions of Compound A -- benzyl 2-[4-(6-chloro-2-quinoxalinyloxy)phenoxy]propanoate, a compound within the scope of U.S. Patent 4,629,493 (Compound 55) -- were prepared and tested.

The structures of the tested compounds are as follows:

Compound Number 2

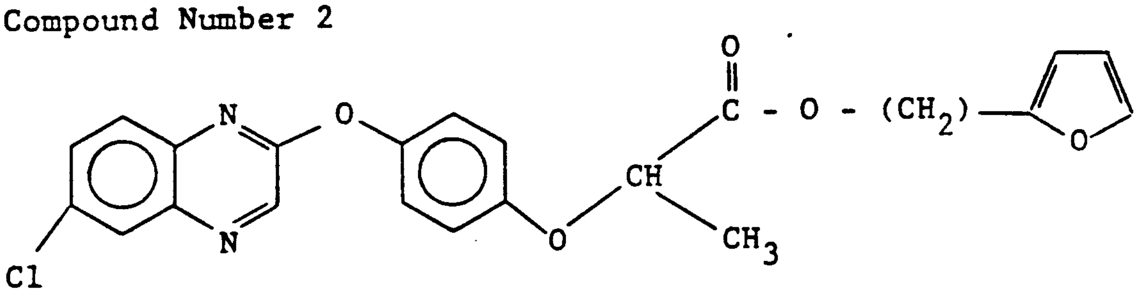

Compound Number 4

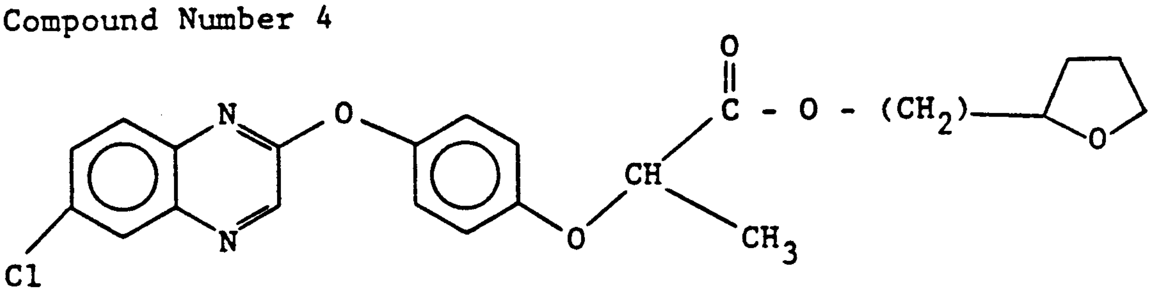

Compound Number 6

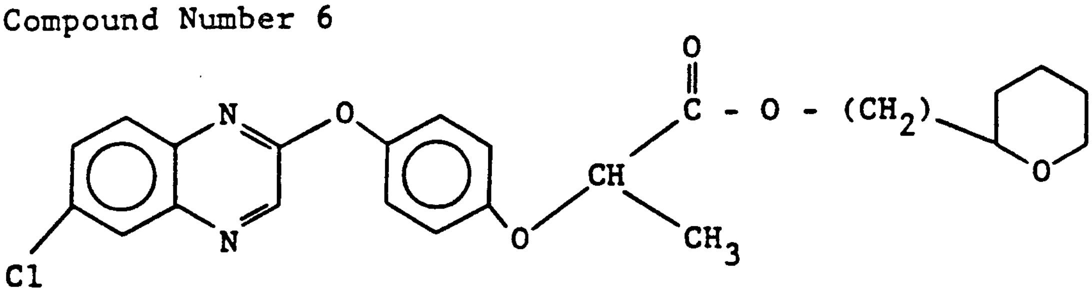

Compound A

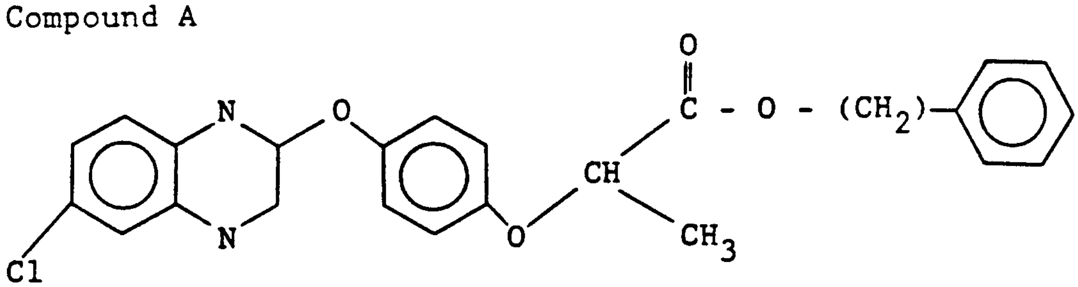

The results of such testing are summarized in Table IV below.

Table IV

| Compound | Rate kg/ha | Percent Weed Control | | |
|---|---|---|---|---|
| | | Wild Oats | Green Foxtail | Barnyard Grass |
| 2 | 0.14 | 60 | 80 | 60 |
| 4 | 0.14 | 70 | 80 | 80 |
| 6 | 0.14 | 50 | 70 | 90 |
| A | 0.14 | 50 | 65 | 30 |

The above data clearly shows the unexpected desirable activity of the compounds of the present invention relative to known homocyclic 5- or 6-membered ring derivatives.

**Claims**

**1.** A compound having the structural formula:

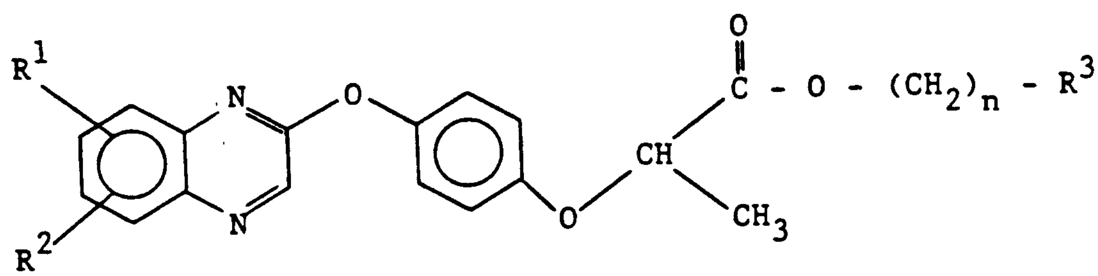

wherein:

n is 1, 2 or 3;

$R^1$ and $R^2$ are each independently selected from the group consisting of halogen, hydrogen, $C_1$-$C_3$ alkoxy, $C_1$-$C_3$ haloalkoxy, monohalomethyl, dihalomethyl, trihalomethyl, cyanato and nitro; and

$R^3$ is a 5- or 6-membered saturated, unsaturated or partially unsaturated ring containing 1 or 2 oxygen atoms; said ring optionally being substituted with between 1 and 3 substituents each independently selected from the group consisting of oxo, $C_1$-$C_3$ alkyl and $C_1$-$C_3$ alkoxy.

**2.** A compound in accordance with claim 1 wherein:

$R^1$ is chlorine, trichloromethyl or trifluoromethyl;

$R^2$ is hydrogen; and

$R^3$ is furanyl, tetrahydrofuranyl, dioxolanyl, tetrahydrodioxolanyl, pyranyl or tetrahydropyranyl; optionally substituted with 1, 2 or 3 moieties selected from the group consisting of oxo and methyl.

**3.** A compound in accordance with claim 1 wherein said compound is selected from the group consisting of:

2-tetrahydrofuranylmethyl 2-[4-(6-chloro-2-quinoxalinyloxy)phenoxy]-propanoate;
2-furanylmethyl 2-[4-(6-chloro-2-quinoxalinyloxy)phenoxy]propanoate;
2-tetrahydropyranylmethyl 2-[4-(6-chloro-2-quinoxalinyloxy)phenoxy]-propanoate;
2-[2-(2-methyl-1,3-dioxolanyl)]ethyl 2-[4-(6-chloro-2-quinoxalinyloxy)-phenoxy]propanoate;
4-(3,3-dimethyl-2-butyrolactonyl)methyl 2-[4-(6-chloro-2-quinoxalinyloxy)-phenoxy]propanoate;
4-(2,2-dimethyl-1,3-dioxolanyl)methyl 2-[4-(6-chloro-2-quinoxalinyloxy)-phenoxy]propanoate.

**4.** A compound in accordance with claim 1 wherein said compound is 2-tetrahydrofuranylmethy 2-[4-6-chloro-2-quinoxalinyloxy)phenoxy]-propanoate.

**5.** A herbicidal composition comprised of:

(A) a compound having the structural formula:

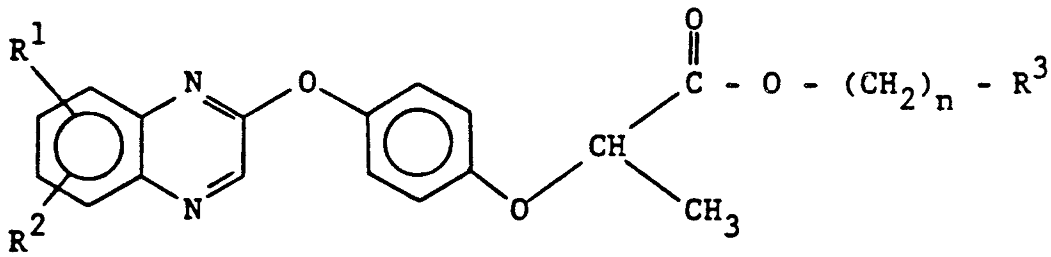

wherein:

n is 1, 2 or 3;

$R^1$ and $R^2$ are each independently selected from the group consisting of halogen, hydrogen, $C_1$-$C_3$ alkoxy, $C_1$-$C_3$ haloalkoxy, monohalomethyl, dihalomethyl, trihalomethyl, cyanato and nitro; and

$R^3$ is a 5- or 6-membered saturated, unsaturated or partially unsaturated ring containing 1 or 2 oxygen atoms; said ring optionally being substituted with between 1 and 3 substituents each independently selected from the group consisting of oxo, $C_1$-$C_3$ alkyl and $C_1$-$C_3$ alkoxy, and

(B) a suitable carrier.

**6.** A composition in accordance with claim 5 wherein, in component (A):

$R^1$ is chlorine, trichloromethyl or trifluoromethyl;

$R^2$ is hydrogen; and

$R^3$ is furanyl, tetrahydrofuranyl, dioxolanyl, tetrahydrodioxolanyl, pyranyl or tetrahydropyranyl.

**7.** A composition in accordance with claim 5 wherein component (A) is selected from the group consisting of:

2-tetrahydrofuranylmethyl 2-[4-(6-chloro-2-quinoxalinyloxy)phenoxy]-propanoate;
2-furanylmethyl 2-[4-(6-chloro-2-quinoxalinyloxy)phenoxy]propanoate;
2-tetrahydropyranylmethyl 2-[4-(6-chloro-2-quinoxalinyloxy)phenoxy]-propanoate;
2-[2-(2-methyl-1,3-dioxolanyl)]ethyl 2 -[4-(6-chloro-2-quinoxalinyloxy)-phenoxy]propanoate;
4-(3,3-dimethyl-2-butyrolactonyl)methyl 2-[4-(6-chloro-2-quinoxalinyloxy)-phenoxy]propanoate; and
4-(2,2-dimethyl-1,3-dioxolanyl)methyl 2-[4-(6-chloro2-quinoxalinyloxy)-phenoxy]propanoate.

**8.** A composition in accordance with claim 5 wherein Compound (A) is 2-tetrahydrofuranylmethyl 2-[4-(6-chloro-2-quinoxalinyloxy)phenoxy]-propanoate.

**9.** A method for controlling the growth of undesirable plants comprising applying to the locus of such undesirable plants a herbicidally effective amount of a compound having the structural formula:

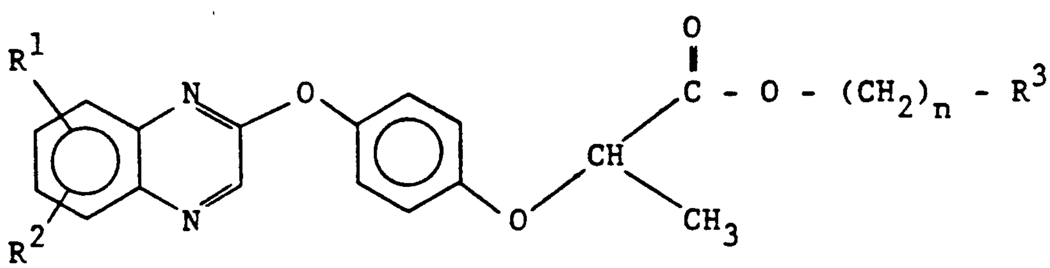

wherein:

n is 1, 2 or 3;

$R^1$ and $R^2$ are each independently selected from the group consisting of halogen, hydrogen, $C_1$-$C_3$ alkoxy, $C_1$-$C_3$ haloalkoxy, monohalomethyl, dihalomethyl, trihalomethyl, cyanato and nitro; and

$R^3$ is a 5- or 6-membered saturated, unsaturated or partially unsaturated ring containing 1 or 2 oxygen atoms; said ring optionally being substituted with between 1 and 3 substituents each independently selected from the group consisting of oxo, $C_1$-$C_3$ alkyl selected from the group consisting of, $C_1$-$C_3$ alkyl and $C_1$-$C_3$ alkoxy.

**10.** A method in accordance with Claim 9 wherein:
$R^1$ is chlorine, trichloromethyl or trifluoromethyl;
$R^2$ is hydrogen; and
$R^3$ is furanyl, tetrahydrofuranyl, dioxolanyl, tetrahydrodioxolanyl, pyranyl or tetrahydropyranyl; optionally substituted with 1, 2 or 3 moieties selected from the group consisting of oxo and methyl.

**11.** A method in accordance with claim 9 wherein said compound is selected from the group consisting of:
2-tetrahydrofuranylmethyl 2-[4-(6-chloro-2-quinoxalinyloxy)phenoxy]-propanoate;
2-furanylmethyl 2-[4-(6-chloro-2-quinoxalinyloxy)phenoxy]propanoate;
2-tetrahydropyranylmethyl 2-[4-(6-chloro-2-quinoxalinyloxy)phenoxy]-propanoate;
2-[2-(2-methyl-1,3-dioxolanyl)]ethyl 2-[4-(6-chloro-2-quinoxalinyloxy)-phenoxy]propanoate;
4-(3,3-dimethyl-2-butyrolactonyl)methyl 2-[4-(6chloro-2-quinoxalinyloxy)-phenoxy]propanoate; and
4-(2,2-dimethyl-1,3-dioxolanyl)methyl 2-[4-(6-chloro-2-quinoxalinyloxy)-phenoxy]propanoate.

**12.** A method in accordance with claim 9 wherein said compound is applied preemergently.

**13.** A method in accordance with claim 12 wherein said compound is 2-tetrahydrofuranylmethyl 2-[4-(6-chloro-2-quinoxalinyloxy)phenoxy]-propanoate.

**14.** A method in accordance with claim 9 wherein said compound is applied postemergently.

**15.** A method in accordance with claim 14 wherein said compound is 2-tetrahydrofuranyl 2-[4-(6-chloro-2-quinoxalinyloxy)phenoxy]-propanoate.

**Patentansprüche**

**1.** Verbindung mit der Strukturformel

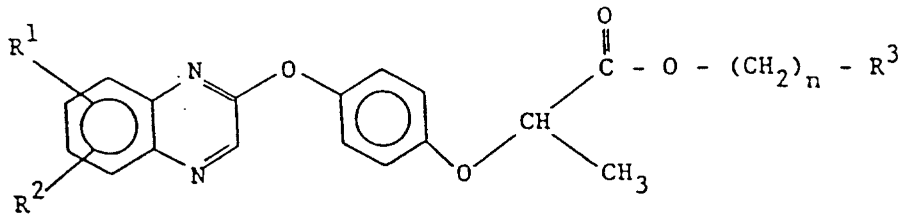

worin
n die Zahl 1, 2 oder 3 ist,
$R^1$ und $R^2$ jeweils unabhängig aus der aus Halogen, Wasserstoff, $C_1$-$C_3$-Alkoxy, $C_1$-$C_3$-Halogenalkoxy, Monohalogenmethyl, Dihalogenmethyl, Trihalogenmethyl, Cyanat und Nitro bestehenden Gruppe ausgewählt sind, und
$R^3$ ein 5- oder 6-gliedriger gesättigter, ungesättigter oder teilweise ungesättigter, 1 oder 2 Sauerstoffatome enthaltender Ring ist, wobei dieser Ring wahlweise mit zwischen 1 und 3 Substituenten substituiert ist, von denen jeder unabhängig aus der aus Oxo, $C_1$-$C_3$-Alkyl und $C_1$-$C_3$-Alkoxy bestehenden Gruppe ausgewählt ist.

**2.** Verbindung nach Anspruch 1, in der
$R^1$ Chlor, Trichlormethyl oder Trifluormethyl ist,
$R^2$ Wasserstoff ist, und
$R^3$ Furanyl, Tetrahydrofuranyl, Dioxolanyl, Tetrahydrodioxolanyl, Pyranyl, oder Tetrahydropyranyl ist, die wahlweise mit 1, 2 oder 3 Teilen substituiert sind, die aus der aus Oxo und Methyl

bestehenden Gruppe ausgewählt sind.

3. Verbindung nach Anspruch 1, die ausgewählt ist aus der Gruppe bestehend aus
2-Tetrahydrofuranylmethyl-2-[4-(6-chlor-2-chinoxalinyloxy)phenoxy]-propanoat,
2-Furanylmethyl-2-[4-(6-chlor-2-chinoxalinyloxy)phenoxy]propanoat,
2-Tetrahydropyranylmethyl-2-[4-(6-chlor-2-chinoxalinyloxy)phenoxy]-propanoat,
2-[2-(2-Methyl-1,3-dioxolanyl)]äthyl-2-[4-(6-chlor-2-chinoxalinyloxy)-phenoxy]propanoat,
4-(3,3-Dimethyl-2-butyrolactonyl)methyl-2-[4-(6-chlor-2-chinoxalinyloxy)-phenoxy]propanoat,
4-(2,2-Dimethyl-1,3-dioxolanyl)methyl-2-[4-(6-chlor-2-chinoxalinyloxy)-phenoxy]propanoat.

4. Verbindung nach Anspruch 1, in der die Verbindung 2-Tetrahydrofuranylmethyl-2-[4-(6-chlor-2-chinoxalinyloxy)phenoxy]-propanoat ist.

5. Herbizide Zusammensetzung aus
(A) einer Verbindung der Strukturformel

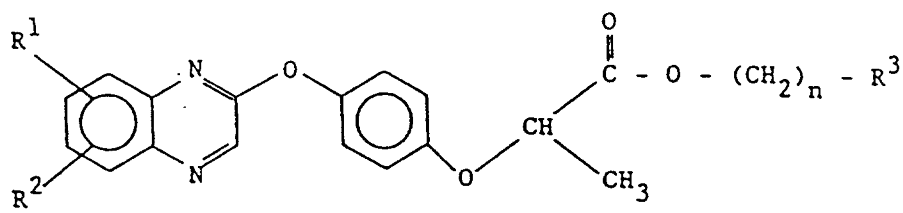

worin

| | |
|---|---|
| n | die Zahl 1, 2 oder 3 ist, |
| $R^1$ und $R^2$ | jeweils unabhängig aus der aus Halogen, Wasserstoff, $C_{1-3}$-Alkoxy, $C_1$-$C_3$-Halogenalkoxy, Monohalogenmethyl, Dihalogenmethyl, Trihalogenmethyl, Cyanat und Nitro bestehenden Gruppe ausgewählt sind, und |
| $R^3$ | ein 5- oder 6-gliedriger gesättigter, ungesättigter oder teilweise ungesättigter, 1 oder 2 Sauerstoffatome enthaltender Ring ist, wobei dieser Ring wahlweise mit zwischen 1 und 3 Substituenten substituiert ist, von denen jeder unabhängig aus der aus Oxo, $C_1$-$C_3$-Alkyl und $C_1$-$C_3$-Alkoxy bestehenden Gruppe ausgewählt ist, und |

(B) einem geeigneten Träger.

6. Zusammensetzung nach Anspruch 5, in der in dem Bestandteil (A)
$R^1$ Chlor, Trichlormethyl oder Trifluormethyl ist,
$R^2$ Wasserstoff ist, und
$R^3$ Furanyl, Tetrahydrofuranyl, Dioxolanyl, Tetrahydrodioxolanyl, Pyranyl oder Tetrahydropyranyl ist.

7. Zusammensetzung nach Anspruch 5, in der der Bestandteil (A) aus der aus
2-Tetrahydrofuranylmethyl-2-[4-(6-chlor-2-chinoxalinyloxy)phenoxy]-propanoat,
2-Furanlymethyl-2-[4-(6-chlor-2-chinocalinyloxy)phenoxy]propanoat,
2-Tetrahydropyranylmethyl-2-[4-(6-chlor-2-chinoxalinyloxy)phenoxy]-propanoat,
2-[2-(2-Methyl-1,3-dioxolanyl)]äthyl-2-[4-(6-chlor-2-chinoxalinyloxy)-phenoxy]-propanoat,
4-(3,3-Dimethyl-2-butyrolactonyl)methyl-2-[4-(6-chlor-2-chinoxalinyloxy)-phenoxy]-propanoat, und
4-(2,2-Dimethyl-1,3-dioxolanyl)methyl-2-[4-(6-chlor-2-chinoxalinyloxy)-phenoxy]-propanoat
bestehenden Gruppe ausgewählt ist.

8. Zusammensetzung nach Anspruch 5, in der die Verbindung (A) 2-Tetrahydrofuranylmethyl-2-[4-(6-chlor-2-chinoxalinyloxy)phenoxy]-propanoat ist.

9. Verfahren zur Wachstumsregelung unerwünschter Pflanzen, bei dem man auf der Stelle dieser unerwünschten Pflanzen eine herbizidisch wirksame Menge einer Verbindung mit der Strukturformel

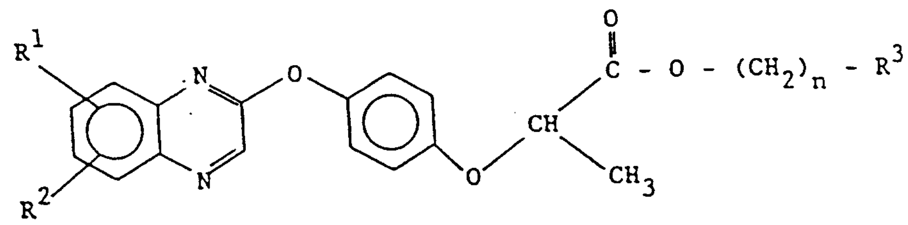

zur Anwendung bringt, worin

n die Zahl 1, 2 oder 3 ist,

$R^1$ und $R^2$ jeweils unabhängig aus der aus Halogen, Wasserstoff, $C_1$-$C_3$-Alkoxy, $C_1$-$C_3$-Halogenalkoxy, Monohalogenmethyl, Dihalogenmethyl, Trihalogenmethyl, Cyanat und Nitro bestehenden Gruppe ausgewählt sind, und

$R^3$ ein 5- oder 6-gliedriger gesättigter, ungesättigter oder teilweise ungesättigter, 1 oder 2 Sauerstoffatome enthaltender Ring ist, wobei dieser Ring wahlweise mit zwischen 1 und 3 Substituenten substituiert ist, von denen jeder unabhängig aus der aus Oxo, $C_1$-$C_3$-Alkyl und $C_1$-$C_3$-Alkoxy bestehenden Gruppe ausgewählt ist.

**10.** Verfahren nach Anspruch 9, bei dem

$R^1$ Chlor, Trichlormethyl oder Trifluormethyl ist,

$R^2$ Wasserstoff ist, und

$R^3$ Furanyl, Tetrahydrofuranyl, Dioxolanyl, Tetrahydrodioxolanyl, Pyranyl oder Tetrahydropyranyl ist, die wahlweise mit 1, 2, oder 3 Teilen substituiert sind, die aus der aus Oxo und Methyl bestehenden Gruppe ausgewählt sind.

**11.** Verfahren nach Anspruch 9, bei dem die Verbindung aus der aus

2-Tetrahydrofuranylmethyl-2-[4-(6-chlor-2-chinoxalinyloxy)phenoxy]-prpanoat,
2-Furanylmethyl-2-[4-(6-chlor-2-chinoxalinyloxy)phenoxy]-propanoat,
2-Tetrahydropyranylmethyl-2-[4-(6-chlor-2-chinoxalinyloxy)phenoxy]-propanoat,
2-[2-(2-Methyl-1,3-dioxolanyl)]äthyl-2-[4-(6-chlor-2-chinoxalinyloxy)-phenoxy]-propanoat,
4-(3,3-Dimethyl-2-butyrolactonyl)methyl-2-[4-(6-chlor-2-chinoxalinyloxy)-phenoxy]-propanoat und
4-(2,2-Dimethyl-1,3-dioxolanyl)methyl-2-[4-(6-chlor-2-chinoxalinyloxy)-phenoxy]-propanoat

bestehenden Gruppe ausgewählt ist.

**12.** Verfahren nach Anspruch 9, bei dem die genannte Verbindung vor dem Auflaufen angewandt wird.

**13.** Verfahren nach Anspruch 12, bei dem die genannte Verbindung 2-Tetrahydrofuranylmethyl-2-[4-(6-chlor-2-chinoxalinyloxy)phenoxy]-propanoat ist.

**14.** Verfahren nach Anspruch 12, bei dem die genannte Verbindung nach dem Auflaufen angewandt wird.

**15.** Verfahren nach Anspruch 14, bei dem die genannte Verbindung 2-Tetrahydrofuranyl-2-[4-(6-chlor-2-chinoxalinyloxy)phenoxy]-propanoat ist.

## Revendications

**1.** Composé ayant la formule de structure :

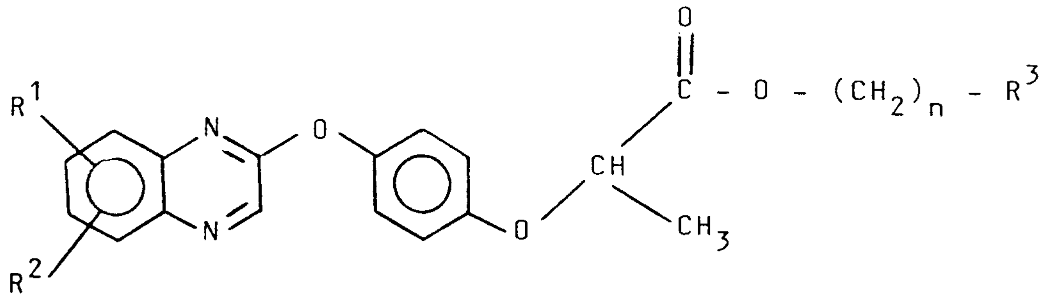

où :

n est 1, 2 ou 3 ;

chacun de

$R^1$ et $R^2$ est indépendamment choisi dans le groupe consistant en halogène, hydrogène, alcoxy $C_1$-$C_3$, haloalcoxy $C_1$-$C_3$, monohalométhyle, dihalométhyle, trihalométhyle, cyanato et nitro ; et

$R^3$ est un noyau hétérocyclique à 5 ou 6 membres saturé, insaturé ou partiellement insaturé contenant 1 ou 2 atomes d'oxygène ; ledit noyau étant facultativement substitué par 1 à 3 substituants, chacun étant indépendamment choisi dans le groupe consistant en oxo, alkyle $C_1$-$C_3$ et alcoxy $C_1$-$C_3$.

**2.** Composé selon la revendication 1, où :

$R^1$ est chlore, trichlorométhyle ou trifluorométhyle ;

$R^2$ est hydrogène ; et

$R^3$ est furanyle, tétrahydrofuranyle, dioxolanyle, tétrahydrodioxolanyle, pyranyle ou tétrahydropyranyle ; facultativement substitué par 1, 2 ou 3 fragments choisis dans le groupe consistant en oxo et méthyle.

**3.** Composé selon la revendication 1, où ledit composé est choisi dans le groupe consistant en :

2-tétrahydrofuranylméthyl 2-[4-(6-chloro-2-quinoxalinyloxy)phénoxy]propanoate ;

2-furanylméthyl 2-[4-(6-chloro-2-quinoxalinyloxy)phénoxy]propanoate ;

2-tétrahydropyranylméthyl 2-[4-(6-chloro-2-quinoxalinyloxy)phénoxy]propanoate ;

2-[2-(2-méthyl-1,3-dioxolanyl)]éthyl 2-[4-(6-chloro-2-quinoxalinyloxy)phénoxy]propanoate ;

4-(3,3-diméthyl-1-butyrolactonyl)methyl 2-[4-(6-chloro-2-quinoxalinyloxy)phénoxy]propanoate ;

4-(2,2-diméthyl-1,3-dioxolanyl)méthyl 2-[4-(6-chloro-2-quinoxalinyloxy)phénoxy]propanoate.

**4.** Composé selon la revendication 1, où ledit composé est 2-tétrahydrofuranylméthyl 2-[4-(6-chloro-2-quinoxalinyloxy)phénoxy]propanoate.

**5.** Composition herbicide composée de :

(A) un composé ayant la formule de structure :

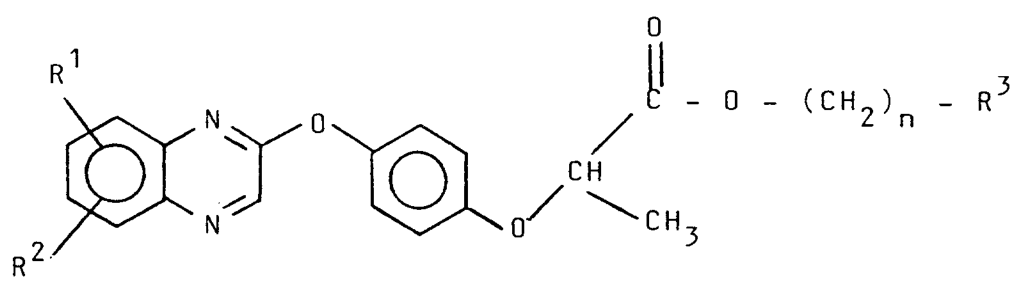

où :

n est 1, 2 ou 3 ;

chacun de

$R^1$ et $R^2$ est indépendamment choisi dans le groupe consistant en halogène, hydrogène, alcoxy $C_1$-$C_3$, haloalcoxy $C_1$-$C_3$, monohalométhyle, dihalométhyle, trihalométhyle, cyanato et nitro ; et

$R^3$ est un noyau hétérocyclique à 5 ou 6 membres saturé, insaturé ou partiellement insaturé, contenant 1 ou 2 atomes d'oxygène ; ledit noyau étant facultativement substitué par 1 à 3 substituants, chacun étant indépendamment choisi dans le groupe consistant en oxo, alkyl $C_1$-$C_3$ et alcoxy $C_1$-$C_3$, et

(B) un support approprié.

**6.** Composition selon la revendication 5 où, dans le composant (A) :

$R^1$ est chlore, trichlorométhyle ou trifluorométhyle ;

$R^2$ est hydrogène ; et

$R^3$ est furanyle, tétrahydrofuranyle, dioxolanyle, tétrahydrodioxolanyle, pyranyle ou tétrahydropyranyle.

**7.** Composition selon la revendication 5, où le composant (A) est choisi dans le groupe consistant en :

2-tétrahydrofuranylméthyl 2-[4-(6-chloro-2-quinoxalinyloxy)phénoxy]propanoate ;

2-furanylméthyl 2-[4-(6-chloro-2-quinoxalinyloxy)phénoxy]propanoate ;

2-tétrahydropyranylméthyl 2-[4-(6-chloro-2-quinoxalinyloxy)phénoxy]propanoate ;

2-[2-(2-méthyl-1,3-dioxolanyl)]éthyl 2-[4-(6-chloro-2-quinoxalinyloxy)phénoxy]propanoate ;

4-(3,3-diméthyl-2-butyrolactonyl)méthyl 2-[4-(6-chloro-2-quinoxalinyloxy)phénoxy]propanoate ; et

4-(2,2-diméthyl-1,3-dioxolanyl)méthyl 2-[4-(6-chloro-2-quinoxalinyloxy)phénoxy]propanoate.

**8.** Composition selon la revendication 5, où le Composé (A) est 2-tétrahydrofuranylméthyl 2-[4-(6-chloro-2-quinoxalinyloxy)phénoxy]propanoate.

**9.** Méthode de contrôle de la croissance des plantes non souhaitables consistant à appliquer, au lieu desdites plantes non souhaitables, une quantité herbicidement efficace d'un composé ayant la formule de structure :

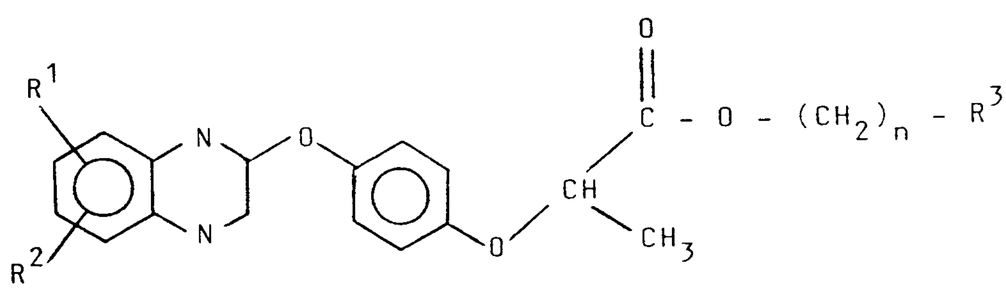

où :

n est 1, 2 ou 3 ;

chacun de

$R^1$ et $R^2$ est indépendamment choisi dans le groupe consistant en halogène, hydrogène, alcoxy $C_1$-$C_3$, haloalcoxy $C_1$-$C_3$, monohalométhyle, dihalométhyle, trihalométhyle, cyanato et nitro ; et

$R^3$ est un noyau hétérocyclique à 5 ou 6 membres saturé, insaturé ou partiellement insaturé, contenant 1 ou 2 atomes d'oxygène ; ledit noyau étant facultativement substitué par 1 à 3 substituants, chacun étant indépendamment choisi dans le groupe consistant en oxo alkyle $C_1$-$C_3$ et alcoxy $C_1$-$C_3$.

**10.** Méthode selon la revendication 9, où

$R^1$ est chlore, trichlorométhyle ou trifluorométhyle ;

$R^2$ est hydrogène ; et

$R^3$ est furanyle, tétrahydrofuranyle, dioxolanyle, tétrahydrodioxolanyle, pyranyle ou tétrahydropyranyle ;

facultativement substitué par 1, 2 ou 3 fragments choisis dans le groupe consistant en oxo et méthyle.

**11.** Méthode selon la revendication 9, où ledit composé est choisi dans le groupe consistant en :
2-tétrahydrofuranylméthyl 2-[4-(6-chloro-2-quinoxalinyloxy)phénoxy]propanoate ;
2-furanylméthyl 2-[4-(6-chloro-2-quinoxalinyloxy)phénoxy]propanoate ;
2-tétrahydropyranylméthyl 2-[4-(6-chloro-2-quinoxalinyloxy)phénoxy]propanoate ;
2-[2-(2-méthyl-1,3-dioxolanyl)]éthyl 2-[4-(6-chloro-2-quinoxalinyloxy)phénoxy]propanoate ;
4-(3,3-diméthyl-2-butyrolactonyl)méthyl 2-[4-(6-chloro-2-quinoxalinyloxy)phénoxy]propanoate ; et
4-(2,2-diméthyl-1,3-dioxolanyl)méthyl 2-[4-(6-chloro-2-quinoxalinyloxy)phénoxy]propanoate.

**12.** Méthode selon la revendication 9, où ledit composé est appliqué avant l'émergence.

**13.** Méthode selon la revendication 12, où ledit composé est le 2-tétrahydrofuranylméthyl 2-[4-(6-chloro-2-quinoxalinyloxy)phénoxy]propanoate.

**14.** Méthode selon la revendication 9, où ledit composé est appliqué après l'émergence.

**15.** Méthode selon la revendication 14, où ledit composé est le 2-tétrahydrofuranyl 2-[4-(6-chloro-2-quinoxalinyloxy)phénoxy]propanoate.